# EUROPÄISCHE PATENTSCHRIFT

(11) **EP 0 460 363 B1**
(45) Veröffentlichungstag und Bekanntmachung des Hinweises auf die Patenterteilung: **02.02.1994**
(21) Anmeldenummer: 91104276.0
(22) Anmeldetag: 20.03.1991
(51) Int. Cl.: C07C 69/33, C07C 67/26

(54) **Verfahren zur Herstellung von Monofettsäure- oder Hydroxymonofettsäureestern von Isopropylidenderivaten eines Polyglycerins**
Method for the production of mono-fatty acid or hydroxy-mono-fatty acid esters of isopropylidene derivatives of a polyglycerol
Procédé pour la fabrication d'esters d'acides gras monovalents ou hydroxyacides gras monovalents de dérivés isopropylidéniques d'un polyglycérol

(30) Priorität: 07.06.1990 DE 4018220
(43) Veröffentlichungstag der Anmeldung: 11.12.1991
(73) Patentinhaber: DEUTSCHE SOLVAY-WERKE GMBH, 42697 Solingen (DE)
(72) Erfinder: Jakobson, Gerald, Dr., W-4134 Rheinberg (DE); Siemanowski, Werner, Dr., W-4134 Rheinberg (DE)
(74) Vertreter: Lauer, Dieter, Dr.

(56) Entgegenhaltungen:
- EP-A- 0 009 904
- EP-A- 0 344 419
- EP-A- 0 380 411
- DE-A- 1 443 596
- PATENT ABSTRACTS OF JAPAN, vol. 11, no. 394 (C-465)(2841), 23 Dezember 1987, & JP-A-62 153255

## Beschreibung

Die vorliegende Erfindung betrifft ein Verfahren zur Herstellung von Monofettsäure- oder Hydroxymonofettsäureestern von Isopropylidenderivaten eines Polyglycerins.

Aus der DE-OS-38 18 292 ist bereits ein Verfahren zur Herstellung von Fettsäure- oder Hydroxyfettsäureestern von Isopropylidenderivaten eines Polyglycerins durch Umsetzung von Fettsäurealkylestern, Mono- oder Polyhydroxyfettsäurealkylestern mit einem oder mehreren Isopropylidenderivaten eines Polyglycerins bekannt. Bei diesem Verfahren ist für die Reaktionsführung ein hoher apparativer Aufwand notwendig, denn zum einen erfolgt die Umsetzung im Vakuum und zum anderen sind mehrere nachfolgende Destillationsstufen zur Abtrennung und Auftrennung der Reaktionsprodukte erforderlich, da die Umsetzung der Ausgangsstoffe zu einem Gemisch von Mono- und Difettsäureestern der eingesetzten Isopropylidenderivate führt.

Aufgabe der vorliegenden Erfindung war es, ein verbessertes Verfahren zur Herstellung von Monofettsäure- oder Hydroxyfettsäureestern von Isopropylidenderivaten eines Polyglycerins zu finden, das bei Normaldruck durchgeführt werden kann und das zu einer möglichst vollständigen Umsetzung der Ausgangsstoffe führt, wobei ausschließlich Monofettsäure- oder Hydroxymonofettsäureester entstehen, so daß eine apparativ aufwendige Abtrennung und Auftrennung der Reaktionsprodukte nicht erforderlich ist.

Die Lösung der Aufgabe wird erfindungsgemäß dadurch erreicht, daß eine C6-C22-Fettsäure oder -Hydroxyfettsäure mit Isopropylidenglyceringlycidylether oder Diisopropylidentriglyceringlycidylether umgesetzt wird, wobei die Ausgangsstoffe in einem Molverhältnis von 0,5:1 bis 1:0,5 eingesetzt werden und die Reaktion in Gegenwart eines Katalysators bei Temperaturen von 373-473 K, vorzugsweise von 403-443 K, erfolgt und der erhaltene Monoisopropylidendiglycerinmonofettsäureester bzw. Monoisopropylidendiglycerinhydroxymonofettsäureester oder Diisopropylidentetraglycerinmonofettsäureester bzw. Diisopropylidentetraglycerinhydroxymonofettsäureester gegebenenfalls durch Destillation, fraktionierte Destillation und/oder Ionenaustausch gereinigt wird.

Die Umsetzung erfolgt bei Normaldruck und führt nach dem erfindungsgemäßen Verfahren ausschließlich zu dem Monofettsäure- oder Hydroxymonofettsäureester in einer Ausbeute von mehr als 95%.

Wird die Reaktion unter 100°C durchgeführt, so sind die Reaktionsgeschwindigkeiten nicht ausreichend hoch, während bei Temperaturen über 200°C ein hoher Prozentsatz unerwünschter Nebenprodukte auftritt.

Als Fettsäuren oder Hydroxyfettsäuren werden gesättigte oder ungesättigte, verzweigte oder unverzweigte Fettsäuren mit C6-C22 eingesetzt, wie zum Beispiel Ester von Vorlauffettsäuren mit C6-C10 oder Behensäure, vorzugsweise jedoch Fettsäuren mit C8-C18 wie beispielsweise Laurinsäure, Myristinsäure, Cocosfettsäuren, Stearinsäure, 2-Ethylhexansäure, Isostearinsäure, Palmölfettsäuren, Ölsäure, Sojaölfettsäuren und/oder Linolsäure. Als C₈-C₁₈-Hydroxyfettsäuren werden beispielsweise 12-Hydroxystearinsäure oder Ricinolsäure eingesetzt.

Als Katalysator wird mindestens ein Ammoniumsalz, ein Phosphoniumsalz und/oder ein tertiäres Amin, vorzugsweise Tetrabutylammoniumbromid, Tetraethylammonimumbromid, Tetrabutylphosphoniumbromid, Tetraethylphosphoniumbromid und/oder Tetramethyl-1,6-Bis-(dimethylamino)-hexan zugefügt.

Nach einer bevorzugten Ausführungsform des erfindungsgemäßen Verfahrens wird der Katalysator in einer Menge von 0,01-5 Gew.-%, vorzugsweise von 0,1-2,5 Gew-%, bezogen auf die Gesamtmenge der umzusetzenden Verbindungen, zugegeben.

Die Umsetzung der Ausgangsstoffe erfolgt zweckmäßig im wasserfreien Zustand, um gewisse Nebenreaktionen auszuschließen.

Nach einer vorzugsweisen Ausführungsform des erfindungsgemäßen Verfahrens erfolgt die Umsetzung der Fettsäure oder der Hydroxyfettsäure mit Isopropylidenglyceringlycidylether oder Diisopropylidentriglyceringlycidylether in einem Molverhältnis von 1:1, wodurch eine nahezu vollständige Umsetzung der Ausgangsstoffe erzielt wird.

Nach einer bevorzugten Ausführungsform wird nach der Umsetzung der Fettsäure oder Hydroxyfettsäure mit Isopropylidenglyceringlycidylether oder Diisopropylidentriglyceringlycidylether der Katalysator durch Destillation des Reaktionsgemisches aus diesem entfernt.

Nach einer weiteren bevorzugten Ausführungsform wird nach der Umsetzung der Ausgangsstoffe der Überschuß an nicht umgesetzter Fettsäure oder Hydroxyfettsäure und/oder nicht umgesetztem Isopropylidenglyceringlycidylether oder Diisopropylidentriglyceringlycidylether im Vakuum abdestilliert. Eine weitere Auftrennung des zurückbleibenden Umsetzungsproduktes ist nicht notwendig.

Die erfindungsgemäß hergestellten Monoisopropylidendiglycerinmonofettsäureester bzw. Monoisopropylidendiglycerinhydroxymonofettsäureester oder Diisopropylidentetraglycerinmonofettsäureester bzw. Diisopropylidentetraglycerinhydroxymonofettsäureester sind als Zwischenprodukte für die Herstellung von nichtionogenen Tensiden geeignet.

Sie können als Lösemittel oder Lösungsvermittler für lipidlösliche oder öllösliche Wirkstoffe sowie in kosmetischen Zubereitungen eingesetzt werden.

### Ausführungsbeispiele

1. Herstellung von Monoisoproylidendiglycerinmonolaurat:
   753 g (4 mol) Isoproylidenglyceringlycidylether und 2,4 g Tetrabutylammoniumbromid werden in einem 2-1-Doppelwandreaktor (Heizflüssigkeit: Öl; Reaktion in Inertgas-Atmosphäre) gegeben und auf ca. 80 °C erwärmt. Unter Rühren werden 800 g (4 mol) Laurinsäure portionsweise zugegeben. Daraufhin wird die Ölvorlauftemperatur auf 150 °C erhöht. Erreicht die Temperatur der Reaktionslösung ca. 140 °C, wird die Ölvorlauftemperatur auf 140 °C gesenkt (übersteigt die Reaktionstemperatur 160 °C, muß gegebenenfalls die Ölvorlauftemperatur kurzzeitig weiter gesenkt werden). Nach ca. 3,5 h ist die Reaktion beendet (Säurezahl: <10, <2% nicht umgesetzter Glycidylether).
   Nicht umgesetzte Edukte werden im Vakuum destillativ entfernt, das Rohprodukt entweder in weiteren Synthesen (z.B. Schutzgruppenabspaltung) eingesetzt oder durch Molekulardestillation rein dargestellt.
   Als Rohprodukt fallen 1,4 kg (ca. 90 % der Theorie) Monoisopropylidendiglycerinmonolaurat an.
2. Herstellung von Diisopropylidentetraglycerinmonolaurat: 752 g (2 mol) Diisopropylidentriglyceringlycidylether und 3,0 g Tetrabutylammoniumbromid werden in einen 2-1-Doppelwandreaktor (Heizflüssigkeit: Öl; Reaktion in Inertgas-Atmosphäre) gegeben und auf ca. 80 °C erwärmt. Unter Rühren werden 400 g (2 mol) Laurinsäure portionsweise zugegeben und anschließend die Ölvorlauftemperatur auf 160 °C erhöht. Nach ca. 3,5 h ist die Reaktionszeit beendet (Säurezahl <10, <2% nicht umgesetzter Glycidylether).
   Nicht umgesetzte Edukte werden durch Molekulardestillation entfernt, das Rohprodukt entweder in weiteren Synthesen (z.B. Schutzgruppenabspaltung) eingesetzt oder durch Molekulardestillation rein dargestellt. Als Rohprodukt fallen 980 g (ca. 85 % der Theorie) Diisopropylidentetraglycerinmonolaurat an.

## Patentansprüche

1. Verfahren zur Herstellung von Monofettsäure- oder Hydroxymonofettsäureestern von Isopropylidenderivaten eines Polyglycerins, dadurch gekennzeichnet, daß eine C6-C22-Fettsäure oder -Hydroxyfettsäure mit Isopropylidenglyceringlycidylether oder Diisopropylidentriglyceringlycidylether in einem Molverhältnis von
0,5:1 bis 1:0,5, vorzugsweise von
1:1,
bei Temperaturen von
373-473 K, vorzugsweise von
403-443 K,
in Gegenwart eines Katalysators umgesetzt und der erhaltene Monoisopropylidendiglycerinmonofettsäureester bzw. Monoisopropylidendiglycerinhydroxymonofettsäureester oder Diisopropylidentetraglycerinmonofettsäureester bzw. Diisopropylidentetraglycerinhydroxymonofettsäureester gegebenenfalls durch Destillation, fraktionierte Destillation und/oder Ionenaustausch gereinigt wird.

2. Verfahren nach Anspruch 1, dadurch gekennzeichnet, daß als Katalysator ein Ammoniumsalz, ein Phosphoniumsalz und/oder ein tertiäres Amin zugefügt wird.

3. Verfahren nach den Ansprüchen 1 und 2, dadurch gekennzeichnet, daß als Katalysator Tetrabutylammoniumbromid, Tetraethylammoniumbromid, Tetrabutylphosphoniumbromid, Tetraethylphosphoniumbromid und/ oder Tetramethyl-1,6-Bis-(dimethylamino)-hexan zugegeben wird.

4. Verfahren nach einem oder mehreren der Ansprüche 1 bis 3, dadurch gekennzeichnet, daß der Katalysator in einer Menge von
0,01-5 Gew.-%, vorzugsweise von
0,1 -2,5 Gew.-%,
bezogen auf die Gesamtmenge der umzusetzenden Verbindungen, zugegeben wird.

5. Verfahren nach einem oder mehreren der Ansprüche 1 bis 4, dadurch gekennzeichnet, daß eine C8-C18-Fettsäure oder -Hydroxyfettsäure eingesetzt wird.

6. Verfahren nach einem oder mehreren der Ansprüche 1 bis 5, dadurch gekennzeichnet, daß nach der Umsetzung der Fettsäure oder Hydroxyfettsäure mit Isopropylidenglyceringlycidylether oder Diisopropylidentriglyceringlycidylether der Katalysator durch Destillation des Reaktionsgemisches aus diesem entfernt wird.

7. Verfahren nach einem oder mehreren der Ansprüche 1 bis 6, dadurch gekennzeichnet, daß nach der Umsetzung der Fettsäure oder Hydroxyfettsäure mit Isopropylidenglyceringlycidylether oder Diisopropylidentriglyceringlycidylether der Überschuß der nicht umgesetzten Fettsäure oder Hydroxyfettsäure und/oder des nicht umgesetzten Isopropylidenglyceringlycidylethers oder Diisopropylidentriglyceringlycidylethers im Vakuum abdestilliert wird.

## Claims

1. A method for producing mono-fatty acid or hydroxy mono-fatty acid esters of isopropylidene derivatives of a polyglycerol, characterised in that a C₆ - C₂₂ fatty acid or hydroxy fatty acid is reacted with isopropylidene glycerol glycidyl ether or diisopropylidene triglycerol glycidyl ether in a molar ratio of
0.5:1 to 1:0.5, preferably of
1:1,
at temperatures of
373-473 K, preferably
403-443 K,
in the presence of a catalyst and the resulting monoisopropylidene diglycerol mono-fatty acid ester or monoisopropylidene diglycerol hydroxy mono-fatty acid ester or diisopropylidene tetraglycerol mono-fatty acid ester or diisopropylidene tetraglycerol hydroxy mono-fatty acid ester is optionally purified by distillation, fractional distillation and/or ion exchange.

2. A method according to Claim 1, characterised in that an ammonium salt, a phosphonium salt and/or a tertiary amine is added as the catalyst.

3. A method according to Claims 1 and 2, characterised in that tetrabutylammonium bromide, tetraethylammonium bromide, tetrabutylphosphonium bromide, tetraethylphosphonium bromide and/or tetramethyl-1,6-bis-(dimethylamino)-hexane is added as the catalyst.

4. A method according to one or more of Claims 1 to 3, characterised in that the catalyst is added in a quantity of
0.01-5% by weight, preferably of
0.1-2.5% by weight,
relative to the total amount of the compounds to be reacted.

5. A method according to one or more of Claims 1 to 4, characterised in that a C8-C18 fatty acid or hydroxy fatty acid is used.

6. A method according to one or more of Claims 1 to 5, characterised in that after the reaction of the fatty acid or hydroxy fatty acid with isopropylidene glycerol glycidyl ether or diisopropylidene triglycerol glycidyl ether the catalyst is removed from the reaction mixture by distillation thereof.

7. A method according to one or more of Claims 1 to 6, characterised in that after the reaction of the fatty acid or hydroxy fatty acid with isopropylidene glycerol glycidyl ether or diisopropylidene triglycerol glycidyl ether the excess of the non-reacted fatty acid or hydroxy fatty acid and/or of the non-reacted isopropylidene glycerol glycidyl ether or diisopropylidene triglycerol glycidyl ether is distilled off in a vacuum.

## Revendications

1. Procédé de production de monoesters d'acides gras ou d'hydroxyacides gras de dérivés isopropylidéniques d'un polyglycérol, caractérisé en ce qu'on fait réagir un acide gras ou un hydroxyacide gras en C₆-C₂₂ avec l'éther glycidylique d'isopropylidène-glycol ou avec l'éther glycidylique de di-isopropylidène triglycérol, selon un rapport molaire de 0,5:1 à 1:0,5, avantageusement de 1:1, à des températures de 373-473K, avantageusement de 403 à 443K en présence d'un catalyseur, et l'on purifie éventuellement par distillation, par distillation fractionnée et/ou par échange d'ions le monoester de l'acide gras de monoisopropylidène diglycérol ou le monoester d'hydroxyacide gras de monoisopropylidène diglycérol ou le monoester de l'acide gras de di-isopropylidène tétraglycérol ou le monoester d'hydroxyacide gras de di-isopropylidène tétraglycérol.

2. Procédé selon la revendication 1, caractérisé en ce qu'on introduit comme catalyseur un sel d'ammonium, un sel de phosphonium et/ou une amine tertiaire.

3. Procédé selon les revendications 1 et 2, caractérisé en ce qu'on ajoute comme catalyseur du bromure de tétrabutyl ammonium, du bromure de tétraéthylammonium, du bromure de tétrabutylphosphonium, du bromure de tétraéthyl- phosphonium et/ou du tétraméthyl-1,6-bis-(diméthylamino)-hexane.

4. Procédé selon une ou plusieurs des revendications 1 à 3, caractérisé en ce qu'on ajoute le catalyseur en une quantité de 0,01 à 5% en poids, avantageusement de 0,1 à 2,5% en poids, par rapport à la quantité totale des composés à faire réagir.

5. Procédé selon une ou plusieurs des revendications 1 à 4, caractérisé en ce qu'on utilise un acide gras ou un hydroxyacide gras en C₈-C₁₈.

6. Procédé selon une ou plusieurs des revendications 1 à 5, caractérisé en ce que, après la réaction de l'acide gras ou de l'hydroxyacide gras avec l'éther glycidylique d'isopropylidène-glycérol ou avec l'éther glycidylique de diisopropylidène triglycérol, on élimine le catalyseur du mélange réactionnel, par distillation de ce mélange.

7. Procédé selon une ou plusieurs des revendications 1 à 6, caractérisé en ce qu'après la réaction de l'acide gras ou de l'hydroxyacide gras avec l'éther glycidylique d'isopropylidène glycérol ou avec l'éther glycidylique de diisopropylidène triglycérol, on chasse par distillation sous vide l'excès de l'acide gras ou de l'hydroxyacide gras n'ayant pas réagi et/ou de l'éther glycidylique d'isopropylidène-glycérol ou de l'éther glycidylique de di-isopropylidène-triglycérol.
